(19) Europäisches Patentamt
European Patent Office
Office européen des brevets

(11) Publication number: **0 068 407**
**A1**

(12) **EUROPEAN PATENT APPLICATION**

(21) Application number: **82105459.0**

(22) Date of filing: **22.06.82**

(51) Int. Cl.³: **C 07 C 143/78**, C 07 C 143/825, C 07 C 143/837, C 07 C 143/79, C 07 D 295/22, C 07 D 237/20, C 07 C 143/80 // A61K31/19, A61K31/235, A61K31/535, A61K31/50, A61K31/495

(30) Priority: **22.06.81 JP 96377/81**
**06.05.82 JP 75781/82**

(43) Date of publication of application: **05.01.83**
**Bulletin 83/1**

(84) Designated Contracting States: **BE IT**

(71) Applicant: **HODOGAYA CHEMICAL CO., LTD., 4-2, Toranomon 1 Chome Minato-ku, Tokyo (JP)**
Applicant: **Mochida Pharmaceutical Co., Ltd., 1-7, Yotsuya Shinjuku-ku, Tokyo (JP)**

(72) Inventor: **Maeda, Hideo, Central Laboratory Hodogaya Chemical Co., Ltd., 6-2-30, Ohji Kita-ku Tokyo (JP)**
Inventor: **Kohno, Shoichi Central Laboratory, Hodogaya Chemical Co. Ltd. 6-2-30, Ohji, Kita-ku Tokyo (JP)**
Inventor: **Ohnishi, Haruo, 6-4-14, Higashi Funabashi, Funabishi-shi Chiba-ken (JP)**

(74) Representative: **Hansen, Bernd, Dr.rer.nat. et al, Hoffmann . Eitle & Partner Patentanwälte Arabellastrasse 4, D-8000 München 81 (DE)**

(54) **Aminosulfonylbenzoic acid derivatives.**

(57) Novel aminosulfonylbenzoic acid derivatives of the formula (I):

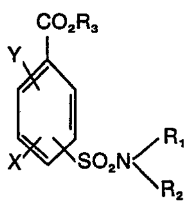

(I)

wherein X and Y, independently of each other, denote chlorine atom, bromine atom, hydrogen atom, amino group or nitro group, $R_1$ denotes hydrogen atom or a lower alkyl group, $R_2$ denotes hydrogen atom, amino group, a lower alkyl group, a hydroxyalkyl group, a lower alkoxy group, aryl group, guanyl group, guanidino group, ureid group, oxamoylamino group, or unsubstituted or halogen-substituted pyridazinoamino group, or $R_1$ and $R_2$ together with a nitrogen atom in the formula (I) may form a saturated heterocyclic group of 4 or 5 carbon atoms in which the carbon atoms may be replaced by an oxygen atom or unsubstituted or lower alkyl–substituted nitrogen atom, and $R_3$ denotes hydrogen atom, alkyl group, alkenyl group, cycloalkyl group, or benzyl group.

EP 0 068 407 A1

37 130 u/wa

# Aminosulfonylbenzoic acid derivatives

The present invention relates to novel aminosulfonylbenzoic acid derivatives of the formula (I):

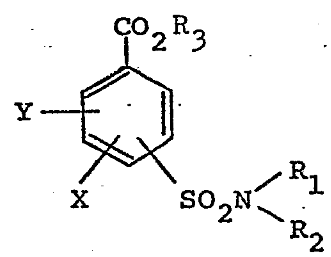

(I)

wherein X and Y, independently of each other, denote chlorine atom, bromine atom, hydrogen atom, amino group or nitro group, $R_1$ denotes hydrogen atom or a lower alkyl group, $R_2$ denotes hydrogen atom, amino group, a lower alkyl group, a hydroxyalkyl group, a lower alkoxy group, aryl group, guanyl group, guanidino group, ureid group, oxamoylamino group, or unsubstituted or halogen-substituted pyridazinoamino group, or $R_1$ and $R_2$ together with a nitrogen atom in the formula (I) may form a saturated



BAD ORIGINAL

heterocyclic group of 4 or 5 carbon atoms in which the carbon atoms may be replaced by an oxygen atom or unsubstituted or lower alkyl group—substituted nitrogen atom, and $R_3$ denotes hydrogen atom, alkyl group, alkenyl group, cycloalkyl group, or benzyl group; and salts of said derivatives.

Upon having multilaterally studied the physiologically active substances, the present inventors have accomplished the present invention on the basis of the acknowledgement that specific aminosulfonylbenzoic acid derivatives have antiviral action.

Therefore, an object of the present invention is to provide novel aminosulfonylbenzoic acid derivatives of the above formula (I) which exhibit antiviral action.

The compounds according to the present invention may be prepared in the following methods.

1. First, chlorosulfonylbenzoic acid is obtained by heating halogenobenzoic acid in chlorosulfonic acid. (GB-PS 896,137) Then, the compounds according to the present invention are prepared by reacting chlorosulfonylbenzoic acid with amines of the formula (II) $NH<^{R_1}_{R_2}$, in which $R_1$ and $R_2$ denote the same meanings as given in the formula (I), in a solvent such as water, dioxane, tetrahydrofuran, chloroform, dichloromethane, dimethoxyethane, benzene, or a mixed solvent of water and one or more of these organic solvents in the presence of an inorganic base such as sodium hydroxide, potassium hydroxide, sodium carbonate, potassium carbonate, sodium bicarbonate, an organic base such as triethylamine, pyridine, diethylaniline or amines of the formula (II) for dehydrochlorination at room temperature, or under heating if necessary.

2. The compounds according to the present invention can be



BAD ORIGINAL

prepared through oxidation of aminosulfonylhalogenotoluene with potassium permanganate or potassium bichromate as oxidizing agent in a solvent such as water, acetone, tert-butylalcohol, chloroform, pyridine, etc.

3. The compounds according to the present invention are also prepared in the following manner.

First, amino-substituted halogenobenzoic acid is converted to diazonium salt in accordance with the ordinary method. Then, sulfurous acid gas is introduced into the reaction mixture, and the resulting reaction mixture is treated with amines of the formula (II), yielding the compounds according to the present invention.

Aminosulfonylhalogenobenzoic ester can be prepared as follows: Halogenoaminosulfonyl benzoic acid is heated in chlorothionyl to obtain halogenoaminosulfonyl benzoic chloride (GB-PS No. 915,259). Halogenoaminosulfonylbenzoic chloride obtained in this way is reacted with an alcohol of the following formula (III):

$$R_4OH \qquad (III)$$

wherein $R_4$ has the same meanings as $R_3$ excluding hydrogen atom. The reaction is performed at room temperature or under heating when necessary in a solvent such as dioxane, tetrahydrofuran, chloroform, dichloromethane, and benzene. Alternatively, the alcohol showing the above formula (III) per se is used as a solvent.



BAD ORIGINAL

The compounds of the present invention thus produced may be used for preparing pharmaceutical preparations, singly or in combination with other active ingredients and, if necessary, with binder, filler and perfume which are usually adopted in the pharmaceutical industry in an appropriate manner known per se.

Examples of the compounds according to the present invention will be described below, but they are merely illustrative of this application and not intended for limiting the scope of the present invention.

Example 1:

2.56 g of 40% an aqueous solution of monomethylamine was dropped into 2.55 g of 3-chlorosulfonyl-4-chlorobenzoic acid suspended in 10 ml of water under stirring and cooling with ice water. After the reaction was effected at room temperature for 2 hours and 10 g of ice was added to the reaction mixture, 6N hydrochloric acid was added thereto to adjust the pH of the reaction mixture to lower than 2, for precipitating crystals. The precipitated crystals were filtered off and washed with water. The crystals were recrystallized from the mixed solvent of water and methanol, yielding 2.03 g of 3-methylaminosulfonyl-4-chlorobenzoic acid as white crystals. The melting point of the product was 236.5 - 239°C when measured in accordance with the prescription of the Japanese Pharmacopeia. The result of elementary analysis of the product is as follows:



BAD ORIGINAL

| | C | H | Cl | N | S |
|---|---|---|---|---|---|
| Calculated | 38.49 | 3.23 | 14.20 | 5.61 | 12.84 |
| Found | 38.28 | 3.21 | 14.15 | 5.69 | 12.99 |

Example 2:

2.22 g of triethylamine and 0.87 g of morphorine were added in these order under stirring into 2.55 g of 3-chlorosulfonyl-4-chlorobenzoic acid dissolved in 10 ml of dioxane while being cooled with ice. After the reaction was effected at room temperature for 1 hour and 30 g of ice water was added to the reaction mixture, it was adjusted with 6 N hydrochloric acid to the pH of lower than 2 for precipitating crystals. The crystals were filtered off and washed with water. Then, the washed crystals was recrystallized from the mixed solvent of water and methanol, yielding 2.34 g of 3-morpholinosulfonyl-4-chlorobenzoic acid as white crystals. In the measurement in accordance with the prescription of the Japanese Pharmacopeia, the product showed the melting point of 187 - 189.5°C. The result of elementary analysis of this product is as follows:

| | C | H | Cl | N | S |
|---|---|---|---|---|---|
| Calculated | 43.21 | 3.96 | 11.60 | 4.58 | 10.49 |
| Found | 43.03 | 3.92 | 11.73 | 4.69 | 10.75 |



BAD ORIGINAL

Example 3:

After 2.55 g of 3-chlorosulfonyl-4-chlorobenzoic acid and 1.94 g of sodium carbonate were dissolved into a solvent consisting of the two phases of 10 ml of water and 10 ml of chloroform, 0.93 g of aniline was dropped therein under stirring and cooling with ice. After 5 hour reaction at room temperature, the reaction mixture was separated into two phases. After addition of 10 g of ice to the aqueous layer, it was adjusted with 6 N hydrochloric acid to the pH of lower than 2 and the precipitated crystals were filtered off and washed with water. The crystals thus obtained were recrystallized from the mixed solvent of water and methanol, yielding 2.65 g of 3-anilinosulfonyl-4-chlorobenzoic acid as white crystals. The product showed the melting point of 208 - 210°C when measured in accordance with the prescription of the Japanese Pharmacopeia. The result of elementary analysis of this product is as follows:

| | C | H | Cl | N | S |
|---|---|---|---|---|---|
| Calculated | 50.09 | 3.23 | 11.37 | 4.49 | 10.28 |
| Found | 50.32 | 3.40 | 11.20 | 4.41 | 10.09 |

Example 4:

1.08 g of guanidine sulfate and 2.90 g of 5-chlorosulfonyl-2,4-dichlorobenzoic acid were successively added into 1.22 g of sodium hydroxide dissolved in 10 ml of water under stirring and cooling with ice. After the reaction was effected at room temperature for 2 hours and then 10 g of ice was added to the reaction mixture, it was adjusted with 6 N hydrochloric acid to the pH of lower than 2 thereby precipitating crystals. The crystals were filtered off and washed with water.

The crystals thus obtained were recrystallized from water, yielding 2.15 g of 5-guanidinosulfonyl-2,4-dichlorobenzoic acid as white crystals. The product showed the melting point of 252-254°C when measured in accordance with the prescription of the Japanese Pharmacopeia. The result of elementary analysis of this product is as follows:

| | C | H | Cl | N | S |
|---|---|---|---|---|---|
| Calculated | 30.78 | 2.26 | 22.72 | 13.46 | 10.27 |
| Found | 30.85 | 2.20 | 22.53 | 13.66 | 10.25 |

Example 5:

After 2.06 g of 2-aminosulfonyl-5-chlorotoluene was dissolved into an aqueous solution of 1.15 g of sodium hydroxide in 10 ml of water, 3.80 g of potassium permanganate was added thereto and the reaction was effected at the temperature of 70 - 80°C for one and a half hours. After excess potassium permanganate was decomposed with a small amount of methanol and the reaction mixture was cooled, the precipitated manganese dioxide was filtered off. The precipitated manganese dioxide was washed with 20 ml of water and the wash liquor was combined with the filtrate and then the solution was adjusted with 6 N hydrochloric acid to the pH of lower than 2. 25 ml of water was distilled off under reduced pressure to precipitate crystals. The crystals were filtered off and recrystallized from water to yield 1.78 g of 2-aminosulfonyl-5-chlorobenzoic acid as white crystals. The product showed the melting point of 162 - 163.5°C when measured in accordance with the prescription of the Japanese Pharmacopeia. The result of elementary analysis of this product is as follows:



BAD ORIGINAL

| | C | H | Cl | N | S |
|---|---|---|---|---|---|
| Calculated | 35.68 | 2.57 | 15.05 | 5.94 | 13.61 |
| Found | 35.50 | 2.55 | 15.25 | 6.04 | 13.35 |

Example 6:

A mixed acid consisting of 125 ml of conc. sulfuric acid and 80 ml of conc. nitric acid was added dropwise to 46 g of 3-chlorosulfonyl-4-chlorobenzoic acid under cooling with ice over a period of 20 minutes. After the temperature was elevated up to 80°C, the reaction was effected under stirring for 8 hours. The reaction mixture was poured into 500 g of ice water and the precipitated crystals was filtered off. After this product was dissolved into 40 ml of 28% ammoniacal water and stirred at room temperature for 2 hours, ammonia was distilled off under reduced pressure. The residue was extracted with ethyl acetate and the ethyl acetate was distilled off from the extract under the reduced pressure after drying the solution over Glauber's salt. The crude product thus obtained was purified by means of the silicagel column chromatography (eluent: chloroform : methanol = 9 : 1) and recrystallization was effected from the mixed solvent of acetone and chloroform, yielding 1.38 g of 3-aminosulfonyl-4-chloro-5-nitrobenzoic acid as white crystals. The product thus obtained showed the melting point of 234 - 236°C when measured in accordance with the prescription of the Japanese Pharmacopeia. The result of elementary analysis of this product is as follows:

| | C | H | Cl | N | S |
|---|---|---|---|---|---|
| Calculated | 29.95 | 1.78 | 12.66 | 9.98 | 11.41 |
| Found | 29.81 | 1.83 | 12.38 | 9.93 | 11.60 |



BAD ORIGINAL

In the following, other examples of the compounds according to the present invention are shown.

Examples 7 - 20

| | Compound Name | Preparation Method * | Appearance | Melting Point (°C) |
|---|---|---|---|---|
| 7 | 3-ethylaminosulfonyl-4-chlorobenzoic acid | 1 | white crystal | 190.5 - 192.5 |
| 8 | 3-dimethylaminosulfonyl-4-chlorobenzoic acid | 1 | ditto | 248.5 - 249.5 |
| 9 | 3-aminosulfonyl-5-bromobenzoic acid | 1 | ditto | 241 - 244 |
| 10 | 3-hydroxyethylaminosulfonyl-4-chlorobenzoic acid | 2 | ditto | 177 - 178 |
| 11 | 3-N-methylhydroxyethylaminosulfonyl-4-chlorobenzoic acid | 2 | ditto | 155.5 - 156.5 |
| 12 | 3-(pyrrolidine-1-yl)sulfonyl-4-chlorobenzoic acid | 2 | ditto | 237 - 239 |
| 13 | 3-isopropylaminosulfonyl-4-chlorobenzoic acid | 3 | ditto | 189 - 191 |
| 14 | 3-methoxyaminosulfonyl-4-chlorobenzoic acid | 4 | ditto | 205 - 206 |
| 15 | 5-guanidinoaminosulfonyl-2,4-dichlorobenzoic acid | 4 | ditto | 198 - 199 (decomposed) |
| 16 | 5-semicarbazidosulfonyl-2,4-dichlorobenzoic acid | 4 | ditto | 220 (decomposed) |
| 17 | 5-(2-oxamoylhydrazinosulfonyl)-2,4-dichlorobenzoic acid | 4 | ditto | 217 - 219 (decomposed) |
| 18 | 2-amino-5-aminosulfonyl-4-chlorobenzoic acid | 1 | ditto | > 300 |
| 19 | 4-chloro-3-[(2-(6-chloropyridazine-3-yl)-hydrazino-1-yl]-sulfonyl-benzoic acid | 2 | ditto | 186 - 188 |
| 20 | 3-(4-methylpyperazine-1-yl) sulfonyl-4-chlorobenzoic acid | 2 | ditto | 120 - 121 |

Note: Numerals 1 - 4 in the column of "Preparation Method" correspond to the above-mentioned Examples 1 - 4.



BAD ORIGINAL

Example 21

Benzyl alcohol of the amount of 1.08 g was added into 2.89 g of 5-aminosulfonyl-2,4-dichlorobenzoic acid chloride dissolved in 10 ml of dioxane. The reaction was performed for 2 hours under reflux. Dioxane was evaporated under reduced pressure and the residue was recrystallized from methanol. As a result, 3.19 g of 5-aminosulfonyl-2,4-dichlorobenzoic benzylester was obtained as white crystals. The melting point of the product was 161.5 - 163 C when measured in accordance with the prescription of the Japanese Pharmacopeia. The result of elementary analysis of the product is as follows:

| | C | H | Cl | N | S |
|---|---|---|---|---|---|
| Calculated | 46.68 | 3.08 | 19.68 | 3.89 | 8.90 |
| Found | 46.40 | 3.01 | 19.39 | 3.92 | 8.75 |



BAD ORIGINAL

Example 22

To 1.5 ml of methanol, 2.38 g of 3-aminosulfonyl-4-fluorobenzoic acid chloride was added. The reaction was performed for 4 hours at room temperature. Methanol was evaporated under reduced pressure and the precipitant was filtered. The product was recrystallized from methanol. As a result, 1.84 g of 3-aminosulfonyl-4-fluorobenzoic methylester was obtained as white crystals. The melting point of the product was 122 - 124°C when measured in accordance with the prescription of the Japanese Pharmacopeia. The result of elementary analysis of the product is as follows:

| | C | H | Cl | N | S |
|---|---|---|---|---|---|
| Calculated | 41.20 | 3.46 | 8.15 | 6.01 | 13.75 |
| Found | 41.40 | 3.41 | 8.10 | 5.92 | 13.65 |

Examples 23-35 as shown in the following according to the present invention were similarly prepared.

| Compound | Chemical Name | Preparation Method* | Appearance | Melting Point (°C) |
|---|---|---|---|---|
| 23 | 3-aminosulfonyl-4-bromo-benzoic isopropylester | 22 | white crystal | 187 - 190 |
| 24 | 3-aminosulfonyl-4-bromo-benzoic n-butylester | 22 | white crystal | 100 - 105 |
| 25 | 5-aminosulfonyl-2,4-dichlorobenzoic methylester | 22 | white crystal | 201.5 - 204 |
| 26 | 5-aminosulfonyl-2,4-dichlorobenzoic ethylester | 22 | white crystal | 124 - 126.5 |
| 27 | 5-aminosulfonyl-2,4-dichloro-benzoic n-propyl ester | 22 | white crystal | 124 - 126.5 |
| 28 | 5-aminosulfonyl-2,4-dichloro-benzoic isopropylester | 22 | white crystal | 149.5 - 151 |
| 29 | 5-aminosulfonyl-2,4-dichloro-benzoic n-butyl ester | 22 | white crystal | 90 - 92 |
| 30 | 5-aminosulfonyl-2,4-dichloro-benzoic sec-butylester | 22 | white crystal | 137.5 - 142 |
| 31 | 5-aminosulfonyl-2,4-dichloro-benzoic n-octylester | 21 | white crystal | 81 - 86 |

| Compound | Chemical Name | Preparation Method* | Appearance | Melting Point (°C) |
|---|---|---|---|---|
| 32 | 5-aminosulfonyl-2,4-dichloro-benzoic cyclohexylester | 22 | white crystal | 130 - 133 |
| 33 | 5-aminosulfonyl-2,4-dichloro-benzoic arylester | 22 | white crystal | 109 - 112 |
| 34 | 5-aminosulfonyl-4-chloro-2-fluorobenzoic methylester | 22 | white crystal | 157 - 161 |
| 35 | 5-aminosulfonyl-2-chloro-4-fluorobenzoic ethylester | 22 | white crystal | 123 - 129 |

Note: numerals 21 and 22 in the column of preparation method correspond to the above mentioned examples 21 and 22 respectively.

The compounds of the present invention possess antiviral activity and especially effective against RNA viruses. They are highly safe in respect of $LD_{50}$ values which are more than 3,000 mg/kg in oral administration and more than 1,000 mg/kg in intraperitoneal injection. Some of them indicated ten times as strong efficacy as that of amanthazine in the test of protective action against the death of mice infected. Highly effective compounds are, for example, compound 24, 29, and 31.



BAD ORIGINAL

Claims:

1. Aminosulfonylbenzoic acid derivatives of the formula (I):

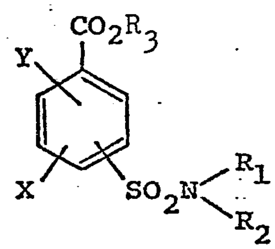

(I)

wherein X and Y, independently of each other, denote chlorine atom, bromine atom, hydrogen atom, amino group or nitro group, $R_1$ denotes hydrogen atom or a lower alkyl group, $R_2$ denotes hydrogen atom, amino group, a lower alkyl group, a hydroxyalkyl group, a lower alkoxy group, aryl group, guanyl group, guanidino group, ureid group, oxamoylamino group, or unsubstituted or halogen-substituted pyridazinoamino group, or $R_1$ and $R_2$ together with a nitrogen atom in the formula (I) may form a saturated heterocyclic group of 4 or 5 carbon atoms in which the carbon atoms may be replaced by an oxygen atom or unsubstituted or lower alkyl—substituted nitrogen atom, and $R_3$ denotes hydrogen atom, alkyl group, alkenyl group, cycloalkyl group, or benzyl group.

2. The aminosulfonylbenzoic acid derivatives according to Claim 1, wherein the compounds of the formula (I) are 3-aminosulfonyl-4-chlorobenzoic acid derivatives.

3. The aminosulfonylbenzoic acid derivatives according to Claim 1, wherein the saturated $C_4$-$C_5$ heterocyclic group formed by $R_1$, $R_2$ and nitrogen atom in the formula (I) in which the carbon atom may be replaced by an oxygen or unsubstituted or lower alkyl substituted nitrogen atom, is one selected from the group



BAD ORIGINAL

consisting of pyrrolidine-1-yl group, morpholine-4-yl group, 4-methylpiperazine-1-yl group, and $R_3$ denotes a hydrogen atom.

4. The aminosulfonylbenzoic acid derivatives according to Claim 1, wherein the compounds of the formula (I) are 5-aminosulfonyl-2,4-dihalogenobenzoic acid derivatives.

5. The aminosulfonylbenzoic acid derivatives according to Claim 1, wherein the compound of the formula (I) is 5-aminosulfonyl-2,4-dichlorobenzoic acid.

6. The aminosulfonylbenzoic acid derivatives according to claim 1 , wherein the compound of the formula (I) is 5-aminosulfonyl-2,4-dichlorobenzoic n-butylester.

7. The aminosulfonylbenzoic acid derivatives according to claim 1, wherein the compound of the formula (I) is 5-aminosulfonyl-2,4-dichlorobenzoic n-octylester.

8. The aminosulfonylbenzoic acid derivatives according to Claim 1, wherein the compound of the formula (I) is 3-aminosulfonyl-5-halogenobenzoic acid.

9. The aminosulfonylbenzoic acid derivatives according to claim 1, wherein the compound of the formula (I) is 3-aminosulfonyl-4-bromobenzoic n-butylester.

10. The aminosulfonylbenzoic acid derivatives according to Claim 1, wherein the compound of the formula (I) is 3-aminosulfonyl-4-chloro-5-nitrobenzoic acid.



BAD ORIGINAL

11. The aminosulfonylbenzoic acid derivatives according to Claim 1, wherein the compound of the formula (I) is 2-amino-5-aminosulfonyl-4-chlorobenzoic acid.

12. The aminosulfonylbenzoic acid derivatives according to Claim 1, wherein the compound of the formula (I) is 3-(4-methylpiperazine-1-yl)sulfonyl-4-chlorobenzoic acid.

13. The aminosulfonylbenzoic acid derivatives according to Claim 1, wherein the compound of the formula (I) is 4-chloro-3-[2-(6-chloropyridazine-3-yl)hydrazino-1-yl]sulfonylbenzoic acid.

14. The aminosulfonylbenzoic acid derivatives according to Claim 1, wherein the compound of the formula (I) is 5-aminosulfonyl-3,4-dihalogenobenzoic acid.



BAD ORIGINAL

0068407

European Patent Office

# EUROPEAN SEARCH REPORT

Application number

EP 82 10 5459.0

| DOCUMENTS CONSIDERED TO BE RELEVANT | | | CLASSIFICATION OF THE APPLICATION (Int. Cl. 3) |
|---|---|---|---|
| Category | Citation of document with indication, where appropriate, of relevant passages | Relevant to claim | |
| X | DE - A - 2 109 339 (MERCK)<br>* claims 1 to 4; examples 1, 2, 4, 5, 8, 9, 12, 16 *<br>-- | 1 | C 07 C 143/78<br>C 07 C 143/825<br>C 07 C 143/837<br>C 07 C 143/79<br>C 07 D 295/22<br>C 07 D 237/20<br>C 07 C 143/80<br>// A 61 K 31/19<br>A 61 K 31/235<br>A 61 K 31/535<br>A 61 K 31/50<br>A 61 K 31/495 |
| X | US - A - 3 088 873 (V. PETROW et al.)<br>* claim 1; example 2 *<br>-- | 1,2 | |
| X | GB - A - 1 353 357 (PFIZER INC.)<br>* page 2, compounds 1 to 20 *<br>-- | 1 | |
| X | GB - A - 1 415 996 (PFIZER INC.)<br>* example 2, compounds 1, 2 *<br>-- | 1 | TECHNICAL FIELDS SEARCHED (Int.Cl. 3)<br>A 61 K 31/00<br>C 07 C 143/00<br>C 07 D 237/20<br>C 07 D 295/22 |
| X | GB - A - 1 458 029 (PFIZER INC.)<br>* example 2, compounds 1, 2; example 5; example 6, compound 2 *<br>-- | 1,4 | |
| X | CH - A - 461 662 (SANDOZ AG)<br>* formula III; example 2, lines 20 to 35, 60 to 67 *<br>-- | 1 | |
| X | US - A - 2 946 815 (G.H. HAMOR)<br>* claims 1, 2; examples 1 to 4, 12 to 16 *<br>---- | 1 | CATEGORY OF CITED DOCUMENTS<br>X: particularly relevant if taken alone<br>Y: particularly relevant if combined with another document of the same category<br>A: technological background<br>O: non-written disclosure<br>P: intermediate document<br>T: theory or principle underlying the invention<br>E: earlier patent document, but published on, or after the filing date<br>D: document cited in the application<br>L: document cited for other reasons |
| X | The present search report has been drawn up for all claims | | &: member of the same patent family, corresponding document |

| Place of search | Date of completion of the search | Examiner |
|---|---|---|
| Berlin | 06-08-1982 | PHILLIPS |